# EUROPÄISCHE PATENTSCHRIFT

(11) **EP 4 509 834 B1**
(45) Veröffentlichungstag und Bekanntmachung des Hinweises auf die Patenterteilung: **02.12.2026**
(21) Anmeldenummer: 23191827.7
(22) Anmeldetag: 17.08.2023
(51) Int. Cl.: G01N 33/28

(54) **VERFAHREN UND VORRICHTUNG ZUM BETREIBEN UND NACHKALIBRIEREN EINES SCHMIERSTOFFSENSORS IN EINEM TECHNISCHEN SYSTEM MIT EINEM SCHMIERSTOFFKREISLAUF**
METHOD AND DEVICE FOR OPERATING AND RE-CALIBRATING A LUBRICANT SENSOR IN A TECHNICAL SYSTEM WITH A LUBRICANT CIRCUIT
PROCÉDÉ ET DISPOSITIF DE FONCTIONNEMENT ET DE POST-ÉTALONNAGE D'UN CAPTEUR DE LUBRIFIANT DANS UN SYSTÈME TECHNIQUE DOTÉ D'UN CIRCUIT DE LUBRIFIANT

(43) Veröffentlichungstag der Anmeldung: 19.02.2025
(73) Patentinhaber: Klüber Lubrication München GmbH & Co. KG, 81379 München (DE)
(72) Erfinder: von der Esch-Letica, Dr. Elisabeth, 81739 München (DE); Fahrnbauer, Dr. Felix, 82319 Starnberg (DE); Nilsson, Martin, 81245 München (DE); Lampe, Günter, 82515 Wolfratshausen (DE); Schmidt, Carolin, 80997 München (DE); Erz, Ruben, 81379 München (DE)
(74) Vertreter: Kuhn, Daniela

(56) Entgegenhaltungen:
- WO-A1-2020/126457
- DE-A1- 102016 005 346
- US-A- 5 968 371
- US-A1- 2010 109 686

## Beschreibung

### Technisches Gebiet

Die Erfindung betrifft Verfahren zum Überwachen eines Schmierstoffs in technischen Systemen mit einer mechanischen Einheit, wie z.B. einem Getriebe, einem Wälzlager und dergleichen. Die Erfindung betrifft weiterhin Maßnahmen zum Sicherstellen einer ordnungsgemäßen sensorischen Erfassung eines Schmierstoffzustands mithilfe eines Schmierstoffsensors.

### Technischer Hintergrund

In technischen Systemen mit mechanischen Einheiten, wie beispielsweise Getrieben, Wälzlagern und dergleichen, wird Schmierstoff in einem Schmierstoffkreislauf zirkuliert, um den Verschleiß von beweglichen Teilen zu verringern, die mechanische Einheit zu reinigen und zu kühlen. Durch den Betrieb des technischen Systems wird Schmierstoff über die Zeit mit Abriebpartikeln und/oder durch thermische Stoffumwandlung und/oder Eindringen oder Kondensation von Wasser verunreinigt und degradiert in seinen Schmiereigenschaften.

Um die Schmierstoffqualität kontinuierlich zu überwachen, sind in der Regel ein oder mehrere Schmierstoffsensoren vorgesehen, die Eigenschaften bzw. den Zustand des Schmierstoffs erfassen. So können beispielsweise Sensoren für eine optische Transparenz, eine Viskosität, eine elektrische Eigenschaft, wie den Ohm'schen Widerstand, die Kapazität und dergleichen, gemessen werden. Durch Überwachung dieser Eigenschaften lässt sich somit die Alterung des Schmierstoffs nachverfolgen und bestimmen, wann dieser ausgetauscht werden muss.

Die Druckschrift WO 2020/126457 A1 offenbart ein Verfahren zum Kalibrieren eines Sensors in einem Kühl-Schmierstoffkreislauf, wobei eine Messung mit frischer Kühl-Schmierstoffemulsion durchgeführt wird, die ein definiertes Verhältnis eines Konzentrats zu Wasser aufweist. Zum Kalibrieren wird der Kreislauf zumindest teilweise entleert, mit der frischen Kühlschmierstoffemulsion gefüllt und eine Referenzmessung durchgeführt. Die sich im Kreislauf befindliche Kühl-Schmierstoffemulsion wird danach in einen Tank zurückgeführt, in dem sich die Gebrauchs-Kühl-Schmierstoffemulsion befindet.

In Druckschrift DE 10 2016 005 346 A1 ist eine Kalibrierung durch Durchströmen des Kalibrierkreislaufs mit Wasser offenbart. Die Durchführung der Referenzmessung bei einem zirkulierenden Wasserkreislauf zeigt eine Kalibriermessung bei einem zirkulierenden Fluid.

Es besteht grundsätzlich das Problem, dass bei einer sensorbasierten Messung aufgrund einer Sensordrift im Laufe der Zeit die Messung der Schmierstoffeigenschaften ungenauer wird, so dass ein hoher Toleranzbereich für die Auswertung der Messwerte und die Bestimmung der Degradation des Schmierstoffs angenommen werden muss. Es ist daher Aufgabe der vorliegenden Erfindung, ein verbessertes Verfahren zum Sicherstellen von korrekten Messwerten eines Schmierstoffsensors zur Verfügung zu stellen, mit dem die Alterung bzw. Degradation des Schmierstoffs besser überwacht werden kann.

### Offenbarung der Erfindung

Diese Aufgabe wird durch das Verfahren zum Nachkalibrieren eines Schmierstoffsensors in einem technischen System mit einem Schmierstoffkreislauf gemäß Anspruch 1 sowie eine Vorrichtung gemäß Anspruch 7 vorgesehen.

Weitere Ausgestaltungen sind in den abhängigen Ansprüchen angegeben.

Gemäß einem ersten Aspekt ist ein Verfahren zum Nachkalibrieren mindestens eines Schmierstoffsensors eines Sensormoduls in einem technischen System mit einer mechanischen Einheit vorgesehen, wobei das Sensormodul in einem Schmierstoffkreislauf mit der mechanischen Einheit genutztem Schmierstoff angeordnet ist, mit folgenden Schritten:
- Entleeren des Schmierstoffkreislaufs, in dem das Sensormodul mit dem mindestens einen Schmierstoffsensor angeordnet ist;
- Zirkulieren von Referenz-Schmierstoff in dem Schmierstoffkreislauf;
- Durchführen einer Referenzmessung mithilfe des mindestens einen Schmierstoffsensors, wenn der Referenz-Schmierstoff das Sensormodul durchströmt;
- Entleeren des Schmierstoffkreislaufs nach der Referenzmessung;
- Füllen des Schmierstoffkreislaufs mit dem von der mechanischen Einheit genutztem Schmierstoff;
- Betreiben des technischen Systems mit dem genutzten Schmierstoff abhängig von dem Ergebnis der Referenzmessung.

In technischen Systemen mit einem Schmierstoffkreislauf werden in der Regel ein oder mehrere Schmierstoffsensoren in einem Sensormodul vorgesehen, um anhand von Messgrößen Eigenschaften bzw. den Zustand des Schmierstoffs zu überwachen. Die Eigenschaften können mithilfe geeigneter Modelle ausgewertet werden, um eine Alterung des Schmierstoffs nachzuverfolgen und einen Alterungszustand des Schmierstoffs zu bestimmen. Diese Modelle können einen einfachen Schwellenwertvergleich vorsehen oder komplexere Funktionen bereitstellen, um ausgehend von den Sensorgrößen den Alterungszustand zu berechnen. Jedoch hängt der Alterungszustand erheblich von der Messgenauigkeit der Schmierstoffsensoren ab.

Erfindungsgemäß ist vorgesehen, dass abhängig von dem Ergebnis der Referenzmessung ein Sensor-Offset des mindestens einen Schmierstoffsensors bestimmt wird, wobei der Sensor-Offset zur Beaufschlagung von Messgrößen des mindestens einen Schmierstoffsensors verwendet wird.

Wie bei vielen Sensoren üblich, unterliegen auch Schmierstoffsensoren einer Sensordrift, die dazu führt, dass der Messgröße während der Lebensdauer eine variierende Offset-Abweichung hinzugefügt wird. Das obige Verfahren sieht dazu vor, mithilfe einer Nachkalibrierung einen Sensor-Offset zu bestimmen, der zum Ausgleichen der Sensordrift dient. Dies ermöglicht ein Rückstellen des Sensorverhaltens auf den Anfangszustand, so dass die verwendeten Modelle zum Bestimmen des Alterungszustands bzw. der Degradation des Schmierstoffs aus den Messgrößen weiterhin genutzt werden können.

Ferner kann vorgesehen sein, dass zum Entleeren des Schmierstoffkreislaufs ein erstes Stellventil und ein zweites Stellventil an einem ersten Ende bzw. einem zweiten Ende des Sensormoduls angeordnet ist, wobei die Trennung des Schmierstoffkreislaufs durch entsprechendes Verstellen der Stellventile erfolgt.

Es ist dazu ein Sensormodul vorgesehen, das mindestens einen Schmierstoffsensor umfasst und dessen Schmierstoffkreislauf mithilfe einer geeigneten Ventilanordnung von einer mechanischen Einheit getrennt werden kann und mit einem Referenz-Schmierstoff, wie z.B. dem gleichen, aber ungenutzten Schmierstoff wie für den Betrieb des mechanischen Systems vorgesehen ist, durchspült werden kann. Dies ermöglicht eine Überwachung der Messwerte des mindestens einen Schmierstoffsensors, wobei die Änderung des Messwerts der aktuellen Referenzmessung zu dem Messwert bei einer Referenzmessung als Sensor-Offset bereitgestellt werden kann, um eine Sensordrift auszugleichen. Die Referenzmessung kann einer Messung von Messwerten bei Inbetriebnahme des Sensormoduls bzw. eines neuen Sensormoduls, zu Beginn der Überwachung des Sensormoduls, bei einem Schmierstoffwechsel oder dergleichen entsprechen.

Zum Durchführen der Referenzmessung wird die Ventilanordnung so gestellt, dass der Schmierstoffkreislauf mit dem Sensormodul und einer Schmierstoffpumpe gebildet wird. Der Schmierstoffkreislauf wird zunächst mithilfe der Schmierstoffpumpe leergepumpt, so dass Leitungen und Sensormodul des Schmierstoffkreislaufs für die Referenzmessung frei von gebrauchtem Schmierstoff sind. Dies kann vorzugsweise erfolgen, indem die Schmierstoffpumpe in Rückwärtsrichtung betrieben wird, um den im Schmierstoffkreislauf befindlichen Schmierstoff zurück in die mechanische Einheit zu befördern.

Das Entleeren des Sensormoduls und der Leitungen im Schmierstoffkreislauf kann durch Betrieb der Schmierstoffpumpe für eine vorbestimmte Zeitdauer erfolgen, oder es kann anhand der Messgrößen des mindestens einen Schmierstoffsensors des Sensormoduls erkannt werden, wenn dieses frei von genutztem Schmierstoff ist und daraufhin die Schmierstoffpumpe abgeschaltet werden.

Weiterhin kann der Referenz-Schmierstoff in einem Referenz-Schmierstoffbehälter aufbewahrt werden, aus dem der Referenz-Schmierstoff zum Durchführen der Referenzmessung entnommen und nach der Referenzmessung wieder zurückgeführt wird.

In einem nachfolgenden Prozessschritt wird nun aus einem Referenz-Schmierstoffbehälter der Referenz-Schmierstoff in den Schmierstoffkreislauf und das Sensormodul gefördert. Dabei wird ein kontinuierlicher, vorbestimmter und konstanter Schmierstoffstrom eingestellt. Somit kann ein kontinuierlicher Durchfluss des Referenz-Schmierstoffs durch das Sensormodul beim Betrieb der Pumpe mit fester Drehzahl oder geregelt basierend auf einem gemessenen Schmierstofffluss eingestellt werden.

Weiterhin kann durch Betreiben einer Heizung der Schmierstoff auf eine gewünschte Messtemperatur für die Referenzmessung erwärmt werden, da manche Sensoren eine Temperaturabhängigkeit haben.

Es wird eine Referenzmessung mit dem mindestens einen Schmierstoffsensor durchgeführt. Dazu werden die Messwerte der Messgrößen in einer Steuereinheit gespeichert.

Alternativ können für die Nachkalibrierung mehrere Referenzmessungen bei verschiedenen vorgegebenen Temperaturen durchgeführt werden, so dass, vorausgesetzt, es wurden initial Referenzmessungen bei den verschiedenen vorgegebenen Temperaturen durchgeführt, für jede der verschiedenen vorgegebenen Temperaturen ein Sensor-Offset bestimmt werden kann. Diese können in einer Lookup-Tabelle abgelegt werden, so dass je nach Betriebstemperatur des technischen Systems ein Sensor-Offset angewendet werden kann, der sich direkt aus der Lookup-Tabelle ergibt oder durch Interpolation daraus ermittelt werden kann.

Nachdem bei einem vorgegebenen Schmierstoffstrom des Referenz-Schmierstoffs und ggf. einer oder mehreren vorgegebenen Temperaturen des Referenz-Schmierstoffs die entsprechenden Messungen mit dem mindestens einen Schmierstoffsensor vorgenommen worden sind, wird der Referenz-Schmierstoff aus dem Schmierstoffkreislauf zurück in den Referenz-Schmierstoffbehälter befördert. Dies erfolgt durch das Betreiben der Schmierstoffpumpe vorzugsweise in Rückwärtsrichtung, bis das Sensormodul und die entsprechenden Leitungen des Teil-Kreislaufs vollständig entleert sind. Anschließend werden die Stellventile der Ventilanordnung wieder so gestellt, dass ein ordnungsgemäßer Betrieb mit dem genutzten Schmierstoff in dem technischen System möglich ist. Dazu wird die Schmierstoffpumpe in Vorwärtsrichtung betrieben, um den genutzten Schmierstoff durch den Schmierstoffkreislauf in die mechanische Einheit des technischen Systems zu befördern.

Das obige Verfahren ermöglicht in besonders effizienter Weise, Schmierstoffsensoren eines Sensormoduls zur Messung von Eigenschaften eines zirkulierenden Schmierstoffs während des Betriebs nachzukalibrieren, ohne das Sensormodul oder einzelne Schmierstoffsensoren ausbauen zu müssen und ohne den genutzten Schmierstoff austauschen zu müssen. Außerdem ist eine Automatisierung des Verfahrens möglich. Es kann weiterhin vorgesehen sein, dass mehrere Referenzmessungen zur Kalibrierung eines jeweiligen Schmierstoffsensors mit verschiedenen Referenz-Schmierstoffen in jeweiligen Referenz-Schmierstoffbehältern durchgeführt werden, aus denen der jeweilige Referenz-Schmierstoff zum Durchführen der entsprechenden Referenzmessung entnommen wird und nach der entsprechenden Referenzmessung wieder zurückgeführt wird.

Gemäß einem weiteren Aspekt ist ein technisches System vorgesehen, umfassend:
- eine mechanische Einheit;
- ein Sensormodul mit mindestens einem Schmierstoffsensor;
- eine Schmierstoffpumpe;
- einen Schmierstoffkreislauf, der ausgebildet ist, um den in der mechanischen Einheit genutzten Schmierstoff durch das Sensormodul mithilfe der Schmierstoffpumpe zu zirkulieren;
- eine Ventilanordnung zum Abtrennen des Schmierstoffkreislaufs, in dem sich das Sensormodul befindet;
- mindestens eine Steuereinheit, die ausgebildet ist, um eine Ansteuerung so vorzunehmen, dass
- zumindest eines Teils des Schmierstoffkreislaufs entleert wird, so dass das Sensormodul mit dem mindestens einen Schmierstoffsensor frei von von der mechanischen Einheit genutztem Schmierstoff ist,
- Referenz-Schmierstoff in dem Schmierstoffkreislauf durch das Sensormodul zirkuliert wird;
- mindestens eine Referenzmessung mithilfe des mindestens einen Schmierstoffsensors durchgeführt wird, wenn der Referenz-Schmierstoff das Sensormodul durchströmt;
- der Schmierstoffkreislauf nach der mindestens einen Referenzmessung entleert wird;
- der Schmierstoffkreislauf mit dem von der mechanischen Einheit genutztem Schmierstoff gefüllt wird; und
- das technische System mit dem genutzten Schmierstoff abhängig von dem Ergebnis der mindestens einen Referenzmessung betrieben wird, wobei abhängig von dem Ergebnis der Referenzmessung ein Sensor-Offset des mindestens einen Schmierstoffsensors bestimmt wird, wobei der Sensor-Offset zur Beaufschlagung von Messgrößen des mindestens einen Schmierstoffsensors verwendet wird.

Die Steuereinheit kann lokal nahe der mechanischen Einheit angeordnet sein und/ oder cloudbasiert entfernt davon angeordnet sein. Insbesondere kann die Steuereinheit zweiteilig ausgebildet sein, wobei ein lokaler Teil die Steuerung der Ventilanordnung und der Schmierstoffpumpe übernimmt und die Auswertung der Referenzmessung cloudbasiert vorgenommen wird.

### Kurzbeschreibung der Zeichnungen

Ausführungsformen werden nachfolgend anhand der beigefügten Zeichnungen näher erläutert. Es zeigen:
- Figur 1: eine schematische Darstellung eines technischen Systems mit einem Schmierstoffkreislauf und einer Anordnung zur Durchführung einer Referenzmessung mindestens eines Schmierstoffsensors; und
- Figur 2: ein Flussdiagramm zur Veranschaulichung des Verfahrens zum Nachkalibrieren mindestens eines Schmierstoffsensors.

### Beschreibung von Ausführungsformen

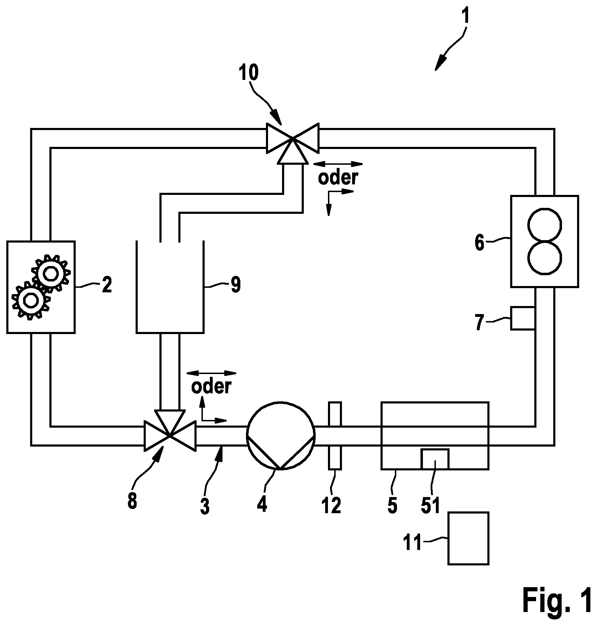

Figur 1 zeigt schematisch ein technisches System 1 mit einer mechanischen Einheit 2, wie z.B. einem Getriebe, und mit einem Schmierstoffkreislauf 3, in dem eine steuerbare Schmierstoffpumpe 4 und ein Sensormodul 5 angeordnet ist. Der Schmierstoffkreislauf 3 verbindet die genannten Elemente fluidisch zum Transport eines in der mechanischen Einheit 2 genutzten Schmierstoffs durch das Sensormodul 5 mithilfe der Schmierstoffpumpe 4.

Das Sensormodul 5 in dem Schmierstoffkreislauf 3 weist mindestens einen Schmierstoffsensor 51 auf. Der mindestens eine Schmierstoffsensor 51 kann einen Viskositätssensor, einen Verschmutzungssensor, einen Kompensationstemperatursensor, einen Leitfähigkeitssensor, einen Permittivitätssensor, einen Transmissionssensor, einen IR Sensor (Mid IR und NIR), einen Sensor für relative Feuchte, einen optischen Partikel-Sensor, einen induktiven Partikel-Sensor, einen Fluoreszenzsensor und dergleichen aufweisen. Die Schmierstoffpumpe 4 steht unmittelbar mit dem Sensormodul 5 in Verbindung.

Ferner kann in dem Schmierstoffkreislauf 3 ein Durchflusssensor 6 und ein Temperatursensor 7 vorgesehen sein. In dem Schmierstoffkreislauf ist ein erstes Stellventil 8 vorgesehen, um den Schmierstoffstrom zu steuern. Das erste Stellventil 8 ist zwischen der Schmierstoffpumpe 4, einem Referenz-Schmierstoffbehälter 9 und der mechanischen Einheit 2 angeordnet.

Ein zweites Stellventil 10 ist zwischen dem Sensormodul 5, der mechanischen Einheit 2 und einem Zulauf des Referenz-Schmierstoffbehälters 9 vorgesehen. In dem Referenz-Schmierstoffbehälter 9 kann sich Referenz-Schmierstoff befinden, wie z.B. ungenutzter Schmierstoff oder mit Additiven versehener ungenutzter Schmierstoff . Das erste und das zweite Stellventil 8, 10 sowie die Schmierstoffsensoren 51 sind mit einer Steuereinheit 11 verbunden.

In einer alternativen Ausführungsform können mehrere, durch ein entsprechendes Ventilsystem separat nutzbare Referenz-Schmierstoffbehälter vorgesehen sein, die jeweils mit ungenutztem Schmierstoff und mit Additiven versehenen ungenutztem Schmierstoff gefüllt sein können. Dies ermöglicht es, nacheinander mehrere Referenzmessungen mit verschiedenen Referenz-Schmierstoffen vorzunehmen. Die Referenz-Schmierstoffe können entsprechend ausgebildet sein, um einen jeweiligen zu kalibrierenden Schmierstoffsensor im Sensormodul zu vermessen.

Die Steuereinheit 11 ist ausgebildet, um ein Verfahren zum Durchführen einer Nachkalibrierung vorzunehmen, um Sensor-Offsets für den mindestens einen Schmierstoffsensor 51 zu ermitteln, mit denen die alterungsbedingte und/oder verschmutzungsbedingte Sensordrift ausgeglichen werden kann. Die Steuereinheit 11 kann lokal und, oder cloudbasiert vorgesehen sein.

Es kann ferner eine Heizung 12 vorgesehen sein, um verschiedene vorbestimmte Temperaturen des Schmierstoffs einzustellen. Somit kann eine Referenzmessung für jeden der Schmierstoffsensoren 51 in dem Sensormodul 5 bei verschiedenen Temperaturen des Schmierstoffs vorgenommen werden, um Referenzwerte zu erhalten. Die jeweiligen Sensor-Offsets ergeben sich somit mit Bezug zu den Referenzwerten einer initialen Referenzmessung für jeden Schmierstoffsensor 51 und für jede der vorbestimmten Temperaturen. Mehrere Sensor-Offsets für einen Schmierstoffsensor 51 bei verschiedenen Temperaturen können in einem Kennfeld oder einer Lookup-Tabelle abgelegt sein, so dass je nach Betriebstemperatur der passende Sensor-Offset angewendet werden kann.

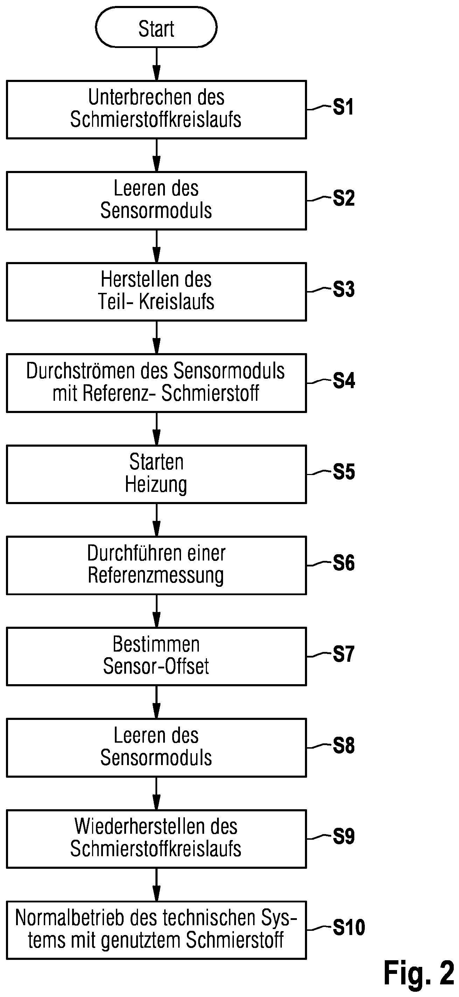

In dem Flussdiagramm der Figur 2 ist das Verfahren zum Durchführen der Nachkalibrierung näher beschrieben. Im Normalbetrieb verbindet in einer ersten Ventilstellung das erste Stellventil 8 die Schmierstoffpumpe 4 mit der mechanischen Einheit 2 und sperrt die Verbindung zu dem Referenz-Schmierstoffbehälter 9. Das zweite Stellventil 10 verbindet in einer ersten Ventilstellung das Sensormodul 5 mit der mechanischen Einheit 2, so dass der Schmierstoffkreislauf geschlossen ist und direkt mit der mechanischen Einheit 2 in Verbindung steht, wobei der Zugang zu dem Referenz-Schmierstoffbehälter 9 gesperrt ist. Die Schmierstoffpumpe 4 befördert den Schmierstoff in einer Vorwärtsrichtung von der mechanischen Einheit 2 über die Schmierstoffpumpe 4 in Richtung des Sensormoduls 5, so dass dort reguläre Messungen von Sensorgrößen vorgenommen werden können.

Zur Durchführung einer Nachkalibrierung wird zunächst in einem optionalen Schritt S1 das erste Stellventil 8 in der ersten Ventilstellung belassen, in der es die Schmierstoffpumpe 4 mit der mechanischen Einheit 2 verbindet. Das zweite Stellventil 10 kann optional von einer Verbindung des Sensormoduls 5 zu der mechanischen Einheit 2 in eine zweite Ventilstellung, d.h. einer Verbindung des Sensormoduls 5 mit dem Referenz-Schmierstoffbehälters 9 und Sperren der Verbindung zu der mechanischen Einheit 2 umgeschaltet werden, wenn nicht sichergestellt werden kann, dass Luft aus der mechanischen Einheit 2 in den Schmierstoffkreislauf eingesaugt wird. Die Verbindung zwischen dem zweiten Stellventil 10 und dem Referenz-Schmierstoffbehälters 9 ist so, dass lediglich ein Zulauf gebildet wird, über den Luft in den Schmierstoffkreislauf gesaugt werden kann

In Schritt S2 wird die Schmierstoffpumpe 4 in Rückwärtsrichtung betrieben, um den Schmierstoff aus dem Sensormodul 5 und den Leitungsbereichen zwischen dem zweiten Stellventil 10 und dem Sensormodul 5 herauszupumpen, bis sich kein genutzter Schmierstoff mehr in den Leitungen zwischen dem ersten und zweiten Stellventil 8, 10 und dem Sensormodul 5 befindet.

Nach dem Entleeren des Sensormoduls wird in Schritt S3 das erste Stellventil 8 in eine zweite Ventilstellung gebracht. In der zweiten Ventilstellung verbindet das erste Stellventil 8 die Schmierstoffpumpe 4 mit dem Referenz-Schmierstoffbehälter 9 und sperrt die Verbindung zu der mechanischen Einheit 2, d.h. die Schmierstoffpumpe 4 ist direkt mit dem Referenz-Schmierstoffbehälter 9 verbunden und von der mechanischen Einheit 2 getrennt. Weiterhin wird das zweite Stellventil 10 in die zweite Ventilstellung geschaltet, so dass das Sensormodul 5 direkt mit dem Referenz-Schmierstoffbehälter 9 verbunden ist, um so einen gesonderten Kreislauf für den Referenz-Schmierstoff zu schließen.

Die Schmierstoffpumpe 4 wird in Schritt S4 in Vorwärtsrichtung betrieben, so dass der Referenz-Schmierstoff den gesonderten Kreislauf mit dem Sensormodul 5 durchströmt.

Die Schmierstoffpumpe 4 wird dabei so betrieben, dass sich ein bestimmter vorgegebener Referenz-Schmierstofffluss in dem Teil-Kreislauf ergibt. Dazu kann die Schmierstoffpumpe 4 mit einer festgelegten Drehzahl oder in geregeltem Betrieb betrieben werden, wobei die Regelgröße als Messgröße des Durchflusssensors 6 bereitgestellt wird.

Weiterhin kann in Schritt S5 die Heizung 12 gestartet werden und die Temperatur des Referenz-Schmierstoffs mit dem Temperatursensor überprüft werden. Insbesondere wird die Temperatur des Referenz-Schmierstoffs auf eine oder mehrere vorgegebene Temperaturen, wie beispielsweise 40°C und 60°C, eingestellt.

Ist die voreingestellte Temperatur erreicht, so wird in Schritt S6 eine Referenzmessung mit dem Sensormodul 5 durchgeführt. Aus der Referenzmessung ergeben sich Sensorwerte des mindestens einen Schmierstoffsensors 51, die dem Referenz-Schmierstoff entsprechen, wie z.B. dem Schmierstoff in Neuzustand.

Durch Abgleich mit einer initialen Referenz-Messgröße, die der jeweiligen Messgröße für neuen ungenutzten Schmierstoff bei Inbetriebnahme des technischen Systems 1 entsprechen kann, wird in Schritt S7 eine Sensordrift als Differenz ermittelt und als Sensor-Offset bei nachfolgenden Messungen mit dem Sensormodul 5 berücksichtigt.

Die Referenzmessungen können bei mehreren Temperaturen durchgeführt werden, so dass sich aus einer initialen Referenz-Messgröße für die entsprechende Temperatur und der aktuellen Referenz-Messgröße der jeweilige Sensor-Offset für die betreffende Temperatur ergibt.

Nach Durchführen der Referenzmessung wird in Schritt S8 die Schmierstoffpumpe 4 in Rückwärtsrichtung betrieben, um den im Kreislauf befindlichen Referenz-Schmierstoff zurück in den Referenz-Schmierstoffbehälter 9 zu fördern. Dies erfolgt so lange, bis der gesamte Teilkreislauf vollständig von Referenz-Schmierstoff entleert ist.

Nach dem Entleeren des Sensormoduls 5 wird in Schritt S9 das erste Stellventil 8 in eine erste Ventilstellung gebracht, in der das erste Stellventil 8 die Schmierstoffpumpe 4 von dem Referenz-Schmierstoffbehälter 9 trennt und die Verbindung zu der mechanischen Einheit 2 wieder herstellt. Weiterhin wird das zweite Stellventil 10 in die erste Ventilstellung geschaltet, so dass das Sensormodul 5 von dem Referenz-Schmierstoffbehälter 9 getrennt ist, um so den Schmierstoff-Kreislauf wieder herzustellen.

Anschließend wird in Schritt S10 das erste Stellventil 8 und das zweite Stellventil 10 auf die ersten Ventilstellungen eingestellt, so dass der sich in der mechanischen Einheit 2 befindliche Schmierstoff mit der Schmierstoffpumpe 4 durch das Sensormodul 5 und zurück über das zweite Stellventil 10 in der mechanischen Einheit 2 gefördert werden kann, um so den Schmierstoffkreislauf für den Normalbetrieb wiederherzustellen.

## Patentansprüche

1. Verfahren zum automatisierbaren Nachkalibrieren mindestens eines Schmierstoffsensors (51) eines Sensormoduls (5) in einem technischen System (1) mit einer mechanischen Einheit (2), wobei das Sensormodul (5) in einem Schmierstoffkreislauf mit von der mechanischen Einheit (2) genutztem Schmierstoff angeordnet ist, mit folgenden Schritten:
- Entleeren (S2) zumindest eines Teils des Schmierstoffkreislaufs (3), so dass das Sensormodul (5) mit dem mindestens einen Schmierstoffsensor (51) frei von von der mechanischen Einheit (2) genutztem Schmierstoff ist;
- Zirkulieren von Referenz-Schmierstoff in dem Schmierstoffkreislauf (3) durch das Sensormodul (5);
- Durchführen mindestens einer Referenzmessung mithilfe des mindestens einen Schmierstoffsensors (51), wenn der Referenz-Schmierstoff das Sensormodul (5) durchströmt;
- Entleeren (S8) des Schmierstoffkreislaufs (3) nach der mindestens einen Referenzmessung;
- Füllen (S10) des Schmierstoffkreislaufs (3) mit von der mechanischen Einheit (2) genutztem Schmierstoff;
- Betreiben (S10) des technischen Systems (1) mit dem genutzten Schmierstoff abhängig von dem Ergebnis der mindestens einen Referenzmessung, wobei abhängig von dem Ergebnis der Referenzmessung ein Sensor-Offset des mindestens einen Schmierstoffsensors (51) bestimmt wird, wobei der Sensor-Offset zur Beaufschlagung von Messgrößen des mindestens einen Schmierstoffsensors (51) verwendet wird.

2. Verfahren nach Anspruch 1, wobei die Referenzmessung bei mehreren vorbestimmten Temperaturen durchgeführt wird, wobei für jede mehreren vorbestimmten Temperaturen und für den mindestens einen Schmierstoffsensor (51) abhängig von dem Ergebnis der Referenzmessung ein Sensor-Offset des mindestens einen Schmierstoffsensors (51) abhängig von einer initialen Referenzmessung bei der betreffenden Temperatur bestimmt wird, wobei die mehreren Sensor-Offsets in einer Lookup-Tabelle oder einer Kennlinie abhängig von der vorbestimmten Temperatur gespeichert werden, wobei der Sensor-Offset zum Beaufschlagen der Messgrößen des mindestens einen Schmierstoffsensors (51) abhängig von der Betriebstemperatur des Schmierstoffes bestimmt wird.

3. Verfahren nach einem der Ansprüche 1 bis 2, wobei zum Entleeren des Schmierstoffkreislaufs (3) ein erstes Stellventil (8) und ein zweites Stellventil (10) an einem ersten Ende bzw. einem zweiten Ende des Sensormoduls (5) angeordnet ist, wobei die Trennung des Schmierstoffkreislaufs (3) von der mechanischen Einheit (2) durch entsprechendes Verstellen der Stellventile (8, 10) erfolgt.

4. Verfahren nach einem der Ansprüche 1 bis 3, wobei der mindestens eine Schmierstoffsensor (51) mindestens einen der folgenden Sensoren umfasst: einen Viskositätssensor, einen Verschmutzungssensor, einen Kompensationstemperatursensor, einen Leitfähigkeitssensor, einen Permittivitätssensor, einen Transmissionssensor, einen IR Sensor (Mid IR und NIR), einen Sensor für relative Feuchte, einen optischen Partikel-Sensor, einen induktiven Partikel-Sensor und einen Fluoreszenzsensor.

5. Verfahren nach einem der Ansprüche 1 bis 4, wobei der Referenz-Schmierstoff in einem Referenz-Schmierstoffbehälter (9) aufbewahrt wird, aus dem der Referenz-Schmierstoff zum Durchführen der Referenzmessung entnommen wird und nach der Referenzmessung wieder zurückgeführt wird.

6. Verfahren nach einem der Ansprüche 1 bis 4, wobei mehrere Referenzmessungen zur Kalibrierung eines jeweiligen Schmierstoffsensors (51) mit verschiedenen Referenz-Schmierstoffen in jeweiligen Referenz-Schmierstoffbehältern (9) durchgeführt werden, aus denen der jeweilige Referenz-Schmierstoff zum Durchführen der entsprechenden Referenzmessung entnommen wird und nach der entsprechenden Referenzmessung wieder zurückgeführt wird.

7. Technisches System (1) umfassend:
- eine mechanische Einheit (2);
- ein Sensormodul (5) mit mindestens einem Schmierstoffsensor (51);
- eine Schmierstoffpumpe (4);
- einen Schmierstoffkreislauf, der ausgebildet ist, um in der mechanischen Einheit genutzter Schmierstoff durch das Sensormodul (5) mithilfe der Schmierstoffpumpe (4) zu zirkulieren;
- eine Ventilanordnung zum Abtrennen des Schmierstoffkreislaufs (3), in dem sich das Sensormodul (5) befindet, von der mechanischen Einheit (2);
- eine Steuereinheit (11), die ausgebildet ist, um eine Ansteuerung so vorzunehmen, dass
∘ zumindest eines Teils des Schmierstoffkreislaufs (3) entleert wird, so dass das Sensormodul (5) mit dem mindestens einen Schmierstoffsensor (51) frei von von der mechanischen Einheit (2) genutztem Schmierstoff ist,
∘ Referenz-Schmierstoff in dem Schmierstoffkreislauf (3) durch das Sensormodul (5) zirkuliert wird;
∘ mindestens eine Referenzmessung mithilfe des mindestens einen Schmierstoffsensors (51) durchgeführt wird, wenn der Referenz-Schmierstoff das Sensormodul (5) durchströmt;
∘ der Schmierstoffkreislauf (3) nach der mindestens einen Referenzmessung entleert wird;
∘ der Schmierstoffkreislauf (3) mit dem von der mechanischen Einheit (2) genutztem Schmierstoff gefüllt wird; und
∘ das technische System (1) mit dem genutzten Schmierstoff abhängig von dem Ergebnis der mindestens einen Referenzmessung betrieben wird, wobei abhängig von dem Ergebnis der Referenzmessung ein Sensor-Offset des mindestens einen Schmierstoffsensors (51) bestimmt wird, wobei der Sensor-Offset zur Beaufschlagung von Messgrößen des mindestens einen Schmierstoffsensors (51) verwendet wird.

## Claims

1. Method for the automatable recalibration of at least one lubricant sensor (51) of a sensor module (5) in a technical system (1) having a mechanical unit (2), wherein the sensor module (5) is arranged in a lubricant circuit with lubricant used by the mechanical unit (2), having the following steps:
- emptying (S2) at least a part of the lubricant circuit (3) such that the sensor module (5) having the at least one lubricant sensor (51) is free of lubricant used by the mechanical unit (2);
- circulating reference lubricant in the lubricant circuit (3) through the sensor module (5);
- carrying out at least one reference measurement using the at least one lubricant sensor (51) when the reference lubricant flows through the sensor module (5);
- emptying (S8) the lubricant circuit (3) after the at least one reference measurement;
- filling (S10) the lubricant circuit (3) with lubricant used by the mechanical unit (2);
- operating (S10) the technical system (1) with the used lubricant depending on the result of the at least one reference measurement, wherein a sensor offset of the at least one lubricant sensor (51) is determined depending on the result of the reference measurement, wherein the sensor offset is used to apply measurement variables of the at least one lubricant sensor (51).

2. Method according to Claim 1, wherein the reference measurement is carried out at multiple predetermined temperatures, wherein a sensor offset of the at least one lubricant sensor (51) is determined depending on an initial reference measurement at the relevant temperature for each of the multiple predetermined temperatures and for the at least one lubricant sensor (51) depending on the result of the reference measurement, wherein the multiple sensor offsets are stored in a lookup table or a characteristic curve depending on the predetermined temperature, wherein the sensor offset for applying the measurement variables of the at least one lubricant sensor (51) is determined depending on the operating temperature of the lubricant.

3. Method according to one of Claims 1 to 2, wherein, to empty the lubricant circuit (3), a first control valve (8) and a second control valve (10) are arranged at a first end and a second end, respectively, of the sensor module (5), wherein the lubricant circuit (3) is separated from the mechanical unit (2) through corresponding adjustment of the control valves (8, 10).

4. Method according to one of Claims 1 to 3, wherein the at least one lubricant sensor (51) comprises at least one of the following sensors: a viscosity sensor, a contamination sensor, a compensation temperature sensor, a conductivity sensor, a permittivity sensor, a transmission sensor, an IR sensor (mid-IR and NIR), a relative humidity sensor, an optical particle sensor, an inductive particle sensor, and a fluorescence sensor.

5. Method according to one of Claims 1 to 4, wherein the reference lubricant is stored in a reference lubricant container (9) from which the reference lubricant is removed for carrying out the reference measurement and is returned again after the reference measurement.

6. Method according to one of Claims 1 to 4, wherein multiple reference measurements for calibrating a respective lubricant sensor (51) with different reference lubricants are carried out in respective reference lubricant containers (9) from which the respective reference lubricant is removed for carrying out the corresponding reference measurement and is returned again after the corresponding reference measurement.

7. Technical system (1) comprising:
- a mechanical unit (2);
- a sensor module (5) having at least one lubricant sensor (51);
- a lubricant pump (4);
- a lubricant circuit which is designed to circulate lubricant used in the mechanical unit through the sensor module (5) using the lubricant pump (4);
- a valve arrangement for separating the lubricant circuit (3) in which the sensor module (5) is located from the mechanical unit (2);
- a control unit (11) which is designed to perform actuation such that
∘ at least a part of the lubricant circuit (3) is emptied such that the sensor module (5) having the at least one lubricant sensor (51) is free of lubricant used by the mechanical unit (2),
∘ reference lubricant is circulated in the lubricant circuit (3) through the sensor module (5);
∘ at least one reference measurement is carried out using the at least one lubricant sensor (51) when the reference lubricant flows through the sensor module (5); ∘ the lubricant circuit (3) is emptied after the at least one reference measurement;
∘ the lubricant circuit (3) is filled with the lubricant used by the mechanical unit (2); and
∘ the technical system (1) is operated with the used lubricant depending on the result of the at least one reference measurement, wherein a sensor offset of the at least one lubricant sensor (51) is determined depending on the result of the reference measurement, wherein the sensor offset is used to apply measurement variables of the at least one lubricant sensor (51).

## Revendications

1. Procédé pour le recalibrage automatisable d'au moins un capteur de lubrifiant (51) d'un module capteur (5) dans un système technique (1) avec une unité mécanique (2), le module capteur (5) étant disposé dans un circuit de lubrifiant avec du lubrifiant utilisé par l'unité mécanique (2), avec des étapes suivantes :
- vidange (S2) d'au moins une partie du circuit de lubrifiant (3) de telle sorte que le module capteur (5) avec l'au moins un capteur de lubrifiant (51) est exempt de lubrifiant utilisé par l'unité mécanique (2) ;
- circulation de lubrifiant de référence dans le circuit de lubrifiant (3) à travers le module capteur (5) ;
- réalisation d'au moins une mesure de référence à l'aide de l'au moins un capteur de lubrifiant (51) lorsque le lubrifiant de référence traverse le module capteur (5) ;
- vidange (S8) du circuit de lubrifiant (3) après l'au moins une mesure de référence ;
- remplissage (S10) du circuit de lubrifiant (3) avec du lubrifiant utilisé par l'unité mécanique (2) ;
- fonctionnement (S10) du système technique (1) avec le lubrifiant utilisé en fonction du résultat de l'au moins une mesure de référence, un décalage de capteur de l'au moins un capteur de lubrifiant (51) étant déterminé en fonction du résultat de la mesure de référence, le décalage de capteur étant utilisé pour appliquer des variables de mesure de l'au moins un capteur de lubrifiant (51).

2. Procédé selon la revendication 1, la mesure de référence étant réalisée à plusieurs températures prédéfinies, pour chacune des plusieurs températures prédéfinies et pour l'au moins un capteur de lubrifiant (51) en fonction du résultat de la mesure de référence, un décalage de capteur de l'au moins un capteur de lubrifiant (51) étant déterminé en fonction d'une mesure de référence initiale à la température concernée, les plusieurs décalages de capteur étant stockés dans une table de référence ou une courbe caractéristique en fonction de la température prédéfinie, le décalage de capteur étant déterminé pour appliquer les variables de mesure de l'au moins un capteur de lubrifiant (51) en fonction de la température de fonctionnement du lubrifiant.

3. Procédé selon l'une des revendications 1 à 2, une première soupape de réglage (8) et une deuxième soupape de réglage (10) étant disposées respectivement sur une première extrémité et une deuxième extrémité du module capteur (5) pour vidanger le circuit de lubrifiant (3), le circuit de lubrifiant (3) étant séparé de l'unité mécanique (2) par un ajustement correspondant des soupapes de réglage (8, 10).

4. Procédé selon l'une des revendications 1 à 3, l'au moins un capteur de lubrifiant (51) comprenant au moins un des capteurs suivants : un capteur de viscosité, un capteur de contamination, un capteur de température de compensation, un capteur de conductivité, un capteur de permittivité, un capteur de transmission, un capteur IR (Mid IR et NIR), un capteur d'humidité relative, un capteur optique de particules, un capteur inductif de particules et un capteur de fluorescence.

5. Procédé selon l'une des revendications 1 à 4, le lubrifiant de référence étant conservé dans un contenant de lubrifiant de référence (9), depuis lequel le lubrifiant de référence est prélevé pour effectuer la mesure de référence et est renvoyé après la mesure de référence.

6. Procédé selon l'une des revendications 1 à 4, plusieurs mesures de référence pour l'étalonnage d'un capteur de lubrifiant (51) respectif avec des lubrifiants de référence différents étant réalisées dans des contenants de lubrifiant de référence(9) respectifs, à partir desquels le lubrifiant de référence respectif est prélevé pour réaliser la mesure de référence correspondante et est renvoyé après la mesure de référence correspondante.

7. Système technique (1) comprenant :
- une unité mécanique (2) ;
- un module capteur (5) avec au moins un capteur de lubrifiant (51) ;
- une pompe de lubrifiant (4) ;
- un circuit de lubrifiant, qui est formé pour faire circuler du lubrifiant utilisé dans l'unité mécanique à travers le module capteur (5) à l'aide de la pompe de lubrifiant (4) ;
- un ensemble de soupapes pour séparer le circuit de lubrifiant (3), dans lequel le module capteur (5) se trouve, de l'unité mécanique (2) ;
- une unité de commande (11) qui est formée pour actionner un pilotage de telle sorte que
∘ au moins une partie du circuit de lubrifiant (3) est vidangée pour que le module capteur (5) avec l'au moins un capteur de lubrifiant (51) soit exempt de lubrifiant utilisé par l'unité mécanique (2),
∘ du lubrifiant de référence circule dans le circuit de lubrifiant (3) à travers le module capteur (5) ;
∘ au moins une mesure de référence est réalisée à l'aide de l'au moins un capteur de lubrifiant (51) lorsque le lubrifiant de référence circule à travers le module capteur (5) ;
∘ le circuit de lubrifiant (3) est vidangé après l'au moins une mesure de référence ;
∘ le circuit de lubrifiant (3) est rempli avec le lubrifiant utilisé par l'unité mécanique (2) ; et
∘ le système technique (1) fonctionne avec le lubrifiant utilisé en fonction du résultat de l'au moins une mesure de référence, un décalage de capteur de l'au moins un capteur de lubrifiant (51) étant déterminé en fonction du résultat de la mesure de référence, le décalage de capteur étant utilisé pour appliquer des variables de mesure de l'au moins un capteur de lubrifiant (51).
